# EUROPEAN PATENT APPLICATION

(11) **EP 2 433 996 A1**
(43) Date of publication of application: **28.03.2012**
(21) Application number: 11765918.5
(22) Date of filing: 05.04.2011
(51) Int. Cl.: C09J 163/02, A61B 1/00, C09J 11/04, C09J 11/06, C09J 133/00, C09J 163/04

(54) **ADHESIVE COMPOSITION AND ENDOSCOPE**

(30) Priority: 09.04.2010 JP 2010090910
(71) Applicant: Olympus Medical Systems Corp., Tokyo 151-0072 (JP)
(72) Inventor: HIRANO, Yuko, Tokyo 192-8512 (JP); NAKAMURA, Mitsuhiro, Tokyo 192-8512 (JP); MATSUMOTO, Jun, Tokyo 192-8512 (JP)
(74) Representative: Schicker, Silvia
(86) International application number: PCT/JP2011/058626
(87) International publication number: WO 2011/126018

(57) **Abstract**

An adhesive composition is provided, which includes a main agent containing a bisphenol A epoxy resin, a phenol novolac epoxy resin, and an acrylic rubber, a curing agent including xylylenediamine and its derivative, and a filler. The filler is spherical silica having an average particle size of 0.5 to 20 µm, and accounts for 22 to 44% by mass of a total amount of the adhesive composition.

## Description

### Technical Field

The present invention relates to an adhesive composition and an endoscope device.

### Background Art

Since an endoscope is inserted into the body cavity or the like, the diameter of the section to be inserted into the body cavity (inserting section) must be as small as possible. Various components are inserted into the inserting section for imparting various functions to the endoscope.

For example, various tubes are inserted into the inserting section of the endoscope. Specific examples of the tube include a forceps tube for drawing a larger treatment tool through the endoscope thereby improving the treatment through the endoscope, a tube for supplying air into the body cavity thereby reducing the burden to the patient, and a tube for washing the lens surface incorporated into the tip hard portion of the inserting section and for washing dirt in the body cavity. These tubes are normally made of, for example, Teflon (registered trademark), olefin, or silicon. The mouth of the tube is fixed on the tip or operating section using an adhesive.

The tip hard portion of the inserting section includes an optical system for observing the inside of the body cavity. The optical system includes a cover lens and a group of lenses including the same, a cover lens for illumination from a light guide, and a group of lenses including the same. These lens groups are fixed on the lens frame or tip hard portion using an adhesive.

In addition, a light guide for transmitting light to the tip end part, and an image guide for transmitting the image to the eyepiece section are inserted into the inserting section. The light guide and image guide include fiber bundles made by binding many fiber wires. The fiber bundles are fixed on the lens frame or tip hard portion using an adhesive.

Furthermore, an electronic endoscope includes, in addition to the above-described tubes and fiber bundles, cables for transmitting the electrical signals, which are emitted from CCDs and others incorporated into the tip hard portion, to the connector section. These CCDs are protected and fixed using an adhesive.

An adhesive is used for bonding of the components of the endoscope, and for finishing of the outer surface. In advance of the finishing using an adhesive, the end of the flexible envelope tube is wound by a thread from the outside, thereby fixing the tube on the inside member. An adhesive is applied to the thread thereby ensuring insertability of the flexible envelope tube and preventing raveling of the thread. In this manner, finishing of the outer surface and fixing of the thread are achieved.

Along with the diversification of the functions of the endoscope, an increasing number of parts are inserted into the inserting section. In order to minimize the diameter of the inserting section, bonding of the components of the endoscope cannot use bolts or screws. For example, as described in Japanese Patent No. 3806635 and JP-A 2002-238834 (KOKAI), epoxy adhesives are frequently used for bonding components.

Medical endoscopes must be completely sterilized because they are inserted into the body cavity of patients. Examples of the sterilants include aldehyde sterilants such as glutaral aldehyde, hydrogen peroxide, peracetic acid, strongly acidic water, alcohols, benzalkonium chloride (ammonium-based), chlorhexidine glucagon (biguanide-based), alkyl diaminoethyl glycine hydrochloride (ampholytic surfactant), phenol-based, iodine-based, sodium hypochlorite-based (chlorine-based), and sodium hydroxide-based sterilants.

An endoscope is reprocessed normally by removing stains by water washing, and then immersing into a chemical solution of a sterilant, or wiping the surface of the endoscope with gauze impregnated with a sterilizing solution. In recent years, in order to securely prevent infection between patients through an endoscope, a higher level of sterilization is required. Therefore, in addition to a peracetic acid aqueous solution and a hydrogen peroxide solution, which are sterilants with high sterilization effect, plasma gas of hydrogen peroxide is increasingly used. Furthermore, autoclave sterilization by saturated water vapor at 135°C and about 233 kPa (2.3 atm) is also used. Among them, sterilization by a hydrogen peroxide solution or plasma gas of hydrogen peroxide and autoclave sterilization are more widely used, because they will not leave harmful residues such as formalin gas or ethylene oxide gas after sterilization, and do not require ventilation facilities.

### Disclosure of Invention

### Technical Problem

However, along with the diversification of sterilization and disinfection methods, more chemical solutions are used. The epoxy adhesives used for bonding the components of endoscopes are subjected to boiling disinfection and autoclave sterilization, and damaged by, for example, hot water, saturated water vapor, active oxygen of peracetic acid, acidic substances of strongly acidic water, or plasma gas of hydrogen peroxide. Examples of the damage include peeling of the bonded surface. In addition, the adhesive layer is thinned by elution or deterioration of the adhesive, which makes it difficult to maintain the performance of the endoscope.

For example, Japanese Patent No. 3806635 describes the modification of an epoxy resin by mixing the epoxy resin with a rubber or plastic, thereby increasing the bond shear strength and bond peel strength of the adhesive. In addition, JP-A 2002-238834 (KOKAI) proposes a combination of a main agent, which contains an epoxy resin mixed with a rubber or plastic, and a specific amine curing agent.

Although adhesives have higher resistance to oxidative degradation, heat aging, and hydrolysis, more strict demands are imposed on sterilization resistance of adhesives. Adhesives are used for the protection and fixing of CCDs, so that deterioration of the adhesive layer can cause image defects. In order to avoid such defects, permeation of water vapor through the adhesive layer is desired to be minimized.

The present invention is intended to provide an adhesive composition which has a viscosity suitable for forming a flawless adhesive layer, and is cured to form an adhesive layer which achieves sufficient durability against various disinfection methods, and has resistance to water vapor permeation.

The present invention is also intended to provide an endoscope device including an adhesive layer bonding the component members or covering the component member, the adhesive layer exhibiting high resistance to sterilization, and being resistant to water vapor permeation.

### Solution to Problem

The solution to the problem is an adhesive composition including a main agent containing a bisphenol A epoxy resin, a phenol novolac epoxy resin, and an acrylic rubber; a curing agent including xylylenediamine and its derivative; and a filler, the filler being spherical silica having an average particle size of 0.5 to 20 µm, and accounting for 22 to 44% by mass of a total amount of the adhesive composition.

An endoscope device according to one aspect of the present invention is **characterized in that** at least two component members are bonded together through an adhesive layer which is formed by curing the aforementioned adhesive composition.

### Advantageous Effects of Invention

The present invention provides an adhesive composition which has a viscosity suitable for forming a flawless adhesive layer, and is cured to form an adhesive layer which achieves sufficient durability against various disinfection methods, and has resistance to water vapor permeation.

The present invention also provides an endoscope device including an adhesive layer bonding the component members or covering the component member, the adhesive layer exhibiting high resistance to sterilization, and having resistance to water vapor permeation.

### Brief Description of Drawings

FIG. 1 is a perspective view showing the schematic structure of the endoscope according to one embodiment of the present invention;
FIG. 2 is a cutaway side view of the envelope tube fixed on the tip of the endoscope; and
FIG. 3 is a front view of the tip end part of the endoscope.

### Best Mode for Carrying Out the Invention

An adhesive composition and endoscope device using the same according to one embodiment of the invention are further described below.

The adhesive composition according to the present embodiment includes a mixture of an epoxy resin and an acrylic rubber as the main agent. The epoxy resin contains a bisphenol A epoxy resin and a phenol novolac epoxy resin.

The acrylic rubber may be used in the form of, for example, a dispersion in the bisphenol A epoxy resin. The acrylic rubber in the form of fine powder is dispersed in the epoxy resin, and forms a so-called sea-island structure. The acrylic rubber increases flexibility of the bonding operation and curing conditions.

The content of acrylic rubber is preferably about 1 to 20% by mass of the total amount of the main agent. The addition of a predetermined amount of acrylic rubber increases the bond shear strength, bond peel strength, and crosslinking density of the cured product, thereby improving its autoclave resistance and chemical resistance. The content of the acrylic rubber is more preferably about 5 to 15% by mass of the total amount of the main agent.

The content of the bisphenol A epoxy resin is preferably about 30 to 98% by mass, and more preferably about 55 to 80% by mass of the total amount of the main agent. The content of the phenol novolac epoxy resin is preferably about 1 to 50% by mass, and more preferably about 15 to 30% by mass of the total amount of the main agent.

The curing agent is a mixture containing xylylenediamine and its derivative. The addition of xylylenediamine and its derivative increases the rate of reaction with the main agent. Specific examples of the xylylenediamine derivative include an alkylene oxide adduct, a glycidyl ester adduct, a glycidyl ether adduct, a Mannich adduct, an acrylonitrile adduct, an epichlorohydrin adduct, and a xylylenediamine trimer.

The content of the xylylenediamine derivative is preferably about 10 to 95% by mass, and more preferably about 30 to 90% by mass of the total amount of the curing agent. The balance of the curing agent is xylylenediamine.

The compounding ratio between the main agent and curing agent is preferably adjusted such that the epoxy groups of the epoxy resin in the main agent are equivalent to the functional groups of the curing agent reacting with the epoxy groups.

As regards the epoxy resin, the molecular weight of one functional group is referred to as the epoxy equivalent weight, and the amine equivalent weight of an amine curing agent is referred to as the active hydrogen equivalent weight. The theoretical compounding ratio is calculated from the epoxy equivalent weight and amine equivalent weight, and is used as a guide for determining the optimum compounding ratio. The optimum compounding ratio is established in consideration of bond strength and other factors.

The compounding ratio (mass ratio) between the main agent and curing agent is preferably from 10:1 to 10:9. When the compounding ratio between the main agent and curing agent is within the specified range, problems such as oxidation deterioration, hydrolysis, softening deterioration by heat, hardening deterioration, brittle fracture, and bond strength degradation are prevented. The compounding ratio between the main agent and curing agent is more preferably from 10:1 to 10:7.

In addition to the main agent and curing agent, the adhesive composition according to the present embodiment includes a specific type of silica as the filler. The silica used herein is spherical silica having an average particle size of 0.5 to 20 µm, and the content is from 22 to 44% by mass with reference to the total amount of the adhesive composition. The average particle size is a volume average particle size, and determined by a common procedure.

The shape of the silica was regarded as spherical based on the observation by an electron microscope.

The silica used herein may be fused silica, and specific examples thereof include spherical fused silica prepared from natural quartz crystal by melting with a burner, and spherical fused silica prepared from natural quartz crystal by melting with a burner.

When the silica contained in the adhesive composition has an average particle size out of the predetermined range, the adhesive layer formed from the composition will not have resistance to water vapor permeation. In addition, when the silica content in the adhesive composition is out of the predetermined range, the adhesive layer formed from the composition will not have resistance to water vapor permeation. When the silica content is too high, the adhesive composition becomes so viscous that workability markedly deteriorates.

Even when the average particle size and content are within the predetermined range, the adhesive composition containing non-spherical silica cannot form an adhesive layer resistant to water vapor permeation. In addition, the adhesive composition becomes so viscous that workability markedly deteriorates.

When spherical silica having a predetermined average particle size is added in a predetermined amount, the adhesive composition has a viscosity suitable for forming a flawless adhesive layer. The adhesive layer formed by curing the adhesive composition is resistant to water vapor permeation, and will not markedly deteriorate in its resistance even after autoclaving or low temperature hydrogen peroxide plasma sterilization.

More specifically, since the adhesive composition according to the present embodiment has a viscosity of 100000 mPa·s or less, it achieves good workability, and forms a flawless adhesive layer. In addition, the cured product obtained by curing the adhesive composition has a tensile shear strength of 25 MPa or more even after autoclave sterilization or low temperature hydrogen peroxide plasma sterilization, and keeps a moisture permeability coefficient of 800 (g.mm/m²·24 hr).

If any one of the conditions of the silica as the filler, such as the shape, average particle size, or content is not fulfilled, the moisture permeability coefficient will not be in the desired range. These findings were made by the inventors for the first time.

In consideration of resistance to autoclave sterilization, the fused silica is preferably natural silica. The average particle size of the silica is preferably from 1 to 10 µm, and the silica content is preferably from 34 to 42% by mass of the total amount of the composition, thereby decreasing the moisture permeability coefficient.

The adhesive composition according to the present embodiment may contain fumed silica in the amount of about 0.1 to 5% by mass of the total amount of the adhesive, thereby improving thixotropy in consideration of workability.

The adhesive composition according to the present embodiment may further contain additives such as catalysts, tackifiers, solvents, flexibilizers, antioxidants, polymerization retarders, surfactants, fungicides, and coloring agents. These additives may be added to the main agent in advance. Alternatively, these additives may be added to the mixture of the main agent and curing agent.

Using the above-described adhesive composition, for example, the components of the endoscope are bonded as follows.

Firstly, a solution containing the main agent and another solution containing the curing agent are mixed at a predetermined ratio, and a predetermined amount of filler is added to the mixture. Subsequently, the mixture thus obtained is applied to the surface of the endoscope components to be bonded using a brush or the like, and these components are joined and fixed. Thereafter, the object is heated at a predetermined temperature for a predetermined period, whereby the endoscope components are firmly bonded together.

The same procedure may be used for sealing of the imaging device of the endoscope, and finishing of the outer surface and fixing of the end of the flexible envelope tube. In addition, the same procedure may be used for forming a mound of adhesive layer around the viewing lens or illuminating lens.

The heating temperature is preferably about 60 to 135°C, though it depends on, for example, the type and compounding ratio of the main agent and curing agent contained in the adhesive composition. When the heating temperature is within this range, the curing reaction proceeds at a practical speed. In addition, the endoscope components with low heat resistance will not cause heat deterioration. The heating time is preferably about 0.5 to 3 hours.

The members bonded using the adhesive composition are not particularly limited as long as they are component members of the endoscope device. For example, the adhesive composition according to the present embodiment may be used to fix the mouths of various tubes, which are inserted into the inserting section of the endoscope device, on the tip of the inserting section or the operating section. In addition, the lens group placed in the tip hard portion of the inserting section may be fixed on the lens frame or tip hard portion. Alternatively, the fiber bundle inserted into the inserting section may be fixed on the lens frame or tip hard portion. The adhesive composition according to the present embodiment may be used for the protection or fixing of a CCD or other device incorporated into the tip hard portion.

When the adhesive composition according to the present embodiment is used for the outer surface finish, insertability is ensured. More specifically, the end of the flexible envelope tube in the inserting section of the endoscope device is wound by a thread from the outside, thereby fixing the tube on the inside member. The adhesive composition is applied to the wound thread, thereby ensuring insertability by outer surface finish and preventing raveling of the thread.

An endoscope device using the adhesive composition according to the present embodiment is described below with reference to the drawings.

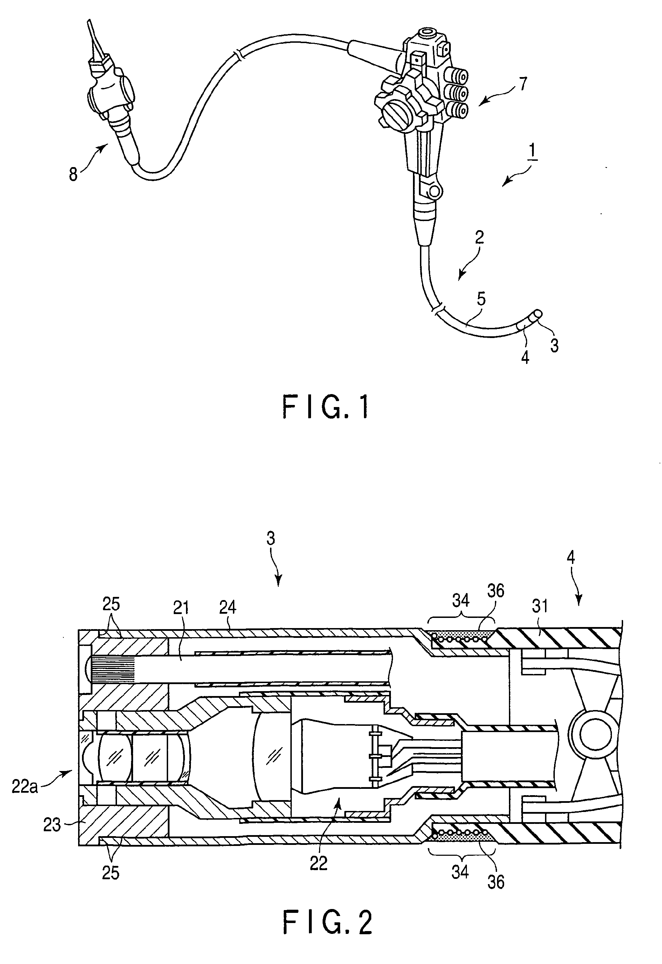

As shown in FIG. 1, the endoscope device 1 according to the present embodiment includes a long slender inserting section 2 to be inserted into the body of the subject, an operating section 7 connected to the inserting section 2, and a universal cord 8 electrically connected to the operating section 7 and supplying illumination light.

The tip end part 3 placed at the tip portion of the inserting section 2 emits illumination light from the tip, and receives reflected light from the inside of the body. A bending part 4 and a flexible tube 5 are bendable and contain optical fibers which transmit light received at the tip end part 3.

In the endoscope device 1, the members to be bonded using the adhesive composition are not particularly limited as long as they are the component members of the endoscope device 1. The mode of use according to the present embodiment is described below with reference to examples.

The tip end part 3 of the endoscope device 1 includes, as shown in FIG. 2, a columnar tip hard portion 23 retaining a light guide fibers 21 supplying illumination light and an imaging unit 22, as well a tip cover 24 which is engaged with a side of the tip hard portion 23. An adhesive layer 25 composed of the above-described adhesive composition is formed at the engaging portion between the tip hard portion 23 and the tip cover 24, thereby bonding them.

A bending tubular rubber 31 covering the outer periphery of the bending part 4 is externally inserted into the end proximal side of the tip cover 24. A thread is wound around the inserted portion of the bending rubber 31 from the outside of the bending rubber 31 to form a thread winding portion 34, thereby fixing the bending rubber 31 on the tip cover 24. An adhesive layer 36 composed of the above-described adhesive composition is formed around the thread winding portion 34, thereby ensuring insertability by the outer surface finish, and preventing raveling of the thread. The adhesive layer 36 covers the thread winding portion 34 along the sides of the tip cover 24 and the bending rubber 31. Upon insertion of the inserting section 2, the tip end part 3 and the bending part 4 are smoothly slid in contact with the living body.

In the endoscope device 1, the adhesive composition may be used to fix the mouths of various tubes, which are inserted into the inserting section 2 of the endoscope device 1, on the tip of the inserting section 2 or the operating section 7. A lens group 22a placed at the tip hard portion 23 of the inserting section 2 may be fixed on the lens frame or the tip hard portion 23. In addition, the fiber bundle inserted into the inserting section 2 may be fixed on the lens frame or the tip hard portion 23.

Furthermore, the adhesive composition may be used for the protection, fixing, or sealing of a CCD of the imaging unit 22 incorporated into the tip end part 3.

Although not shown, the outer periphery of the junction between the bending part 4 and the flexible tube 5 has the same structure as the outer periphery of the junction between the tip end part 3 and the bending part 4. More specifically, a thread winding portion is formed at the junction between the bending part 4 and the flexible tube 5, and the same adhesive composition as that described above is applied to the periphery of the thread winding portion. The adhesive layer composed of the adhesive composition ensures insertability by outer surface finish, and prevents raveling of thread.

The adhesive composition may be used for sealing of the image pickup device in the endoscope device. Furthermore, the adhesive composition may be heaped up around the viewing lens or illuminating lens in the endoscope device, thereby smoothing the edges around the lenses.

The adhesive composition according to the present embodiment may be placed around the lens frame in the tip end part 3 of the endoscope device 1.

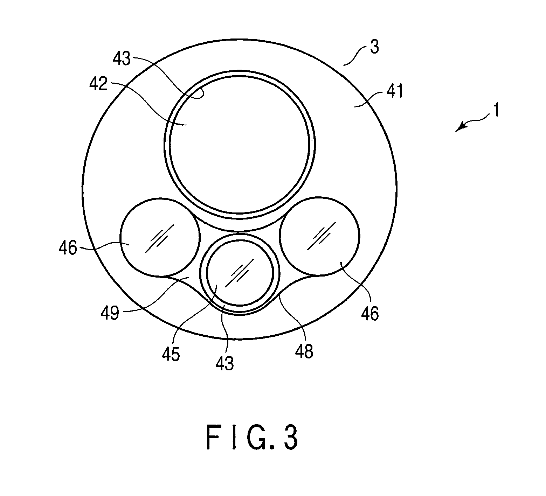

FIG. 3 is a front view of the tip end part 3 of the endoscope 1. An electrical insulating member 41 includes a forceps channel 42, and an objective lens frame 43 is placed on the inner wall. An objective lens 45 is placed between two illuminating lenses 46, and the space between the illuminating lenses 46 and the objective lens frame 43 is filled with an adhesive 49 to form a septum 48. As a result of this, direct incidence of light from the illuminating lenses 46 into the objective lens 45 is prevented, and the illuminating lenses 46 and the objective lens frame 43 are fixed by the adhesive layer 49.

As described above, according to the present embodiment, the adhesive composition includes a main agent containing a bisphenol A epoxy resin, a phenol novolac epoxy resin, and an acrylic rubber, and a curing agent containing xylylenediamine and its derivative, and a filler, the filler being spherical silica having an average particle size of 0.5 to 20 µm, and the content of the silica being from 22 to 44% by mass of the adhesive composition.

The adhesive composition has a viscosity suitable for bonding the endoscope members and finishing the outer surface. The adhesive composition is used for various applications. Examples of the application include bonding of endoscope components, outer surface finish and fixing of thread for the end of the flexible envelope tube in the inserting section of the endoscope, sealing of the image pickup device of the endoscope, or smoothing of edges of the outer periphery of lenses by heaping up the adhesive around the viewing lens or illuminating lens of the endoscope. As a result of this, there is obtained an endoscope device including an adhesive layer which is resistant to water vapor permeation, and will not be deteriorated in its sterilization resistance by various disinfection methods.

### <Examples>

The examples of the present invention are described below, but the present invention will not be limited by the following examples.

### (Example 1)

A bisphenol A epoxy resin, a phenol novolac epoxy resin, and an acrylic rubber were mixed to prepare a main agent. The percentages by mass of these components in the main agent are as follows.

| | |
|---|---|
| Bisphenol A epoxy resin: | 60% by mass |
| Phenol novolac epoxy resin: | 30% by mass |
| Acrylic rubber particles: | 10% by mass |

Subsequently, xylylenediamine and its derivative, or a glycidyl ester adduct were mixed to prepare a curing agent. The percentages by mass of these components in the main agent are as follows.

| | |
|---|---|
| Xylylenediamine: | 10% by mass |
| Xylylenediamine derivative: | 90% by mass |

The main agent and curing agent prepared as described above were mixed at a mass ratio of 10:4, and a filler was added to the mixture thus obtained, thereby producing an adhesive composition No. 1. The filler was spherical fused silica having an average particle size of 4 µm, and the content was 22% by mass of the total amount of the composition.

Subsequently, the adhesive compositions No. 2 to No. 14 were obtained in the same manner as No. 1, except that the average particle size and content of silica as filler were changed as shown in Table 1. The silica used in No. 2 to No. 13 was spherical fused silica, while the silica used in No. 14 was planar.

### [Table 1]

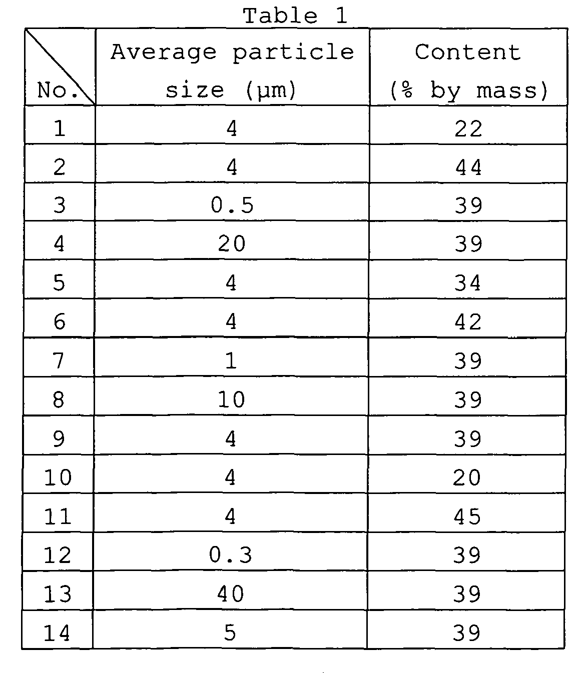

The viscosity of the adhesive compositions thus obtained was measured using a viscoelasticity meter. The viscosity must be 100000 mPa·s or less.

Further, using these adhesive compositions, stainless steel test pieces were bonded by curing at 80°C for 2 hours. The bonded test pieces were measured for tensile shear strength. The tensile shear strength was measured in accordance with JIS K6850 [Tensile-Shear Bond Strength Test for Adhesives]. For the measurement of tensile strength in the autoclave test, firstly, the bonded test pieces were sterilized with water vapor at 135°C using a steam sterilization apparatus. Thereafter, the test pieces were taken out into the air and dried at normal temperature, and measured for the tensile strength after 24 hours. The acceptable shear strength is 25 MPa or more.

The moisture permeability coefficient was determined by an improved method of JIS Z0208 [Cup Method]. The specific procedure is as follows. Firstly, an adhesive composition is cured to make a sample plate. Aside from this, a cup containing a predetermined mass of calcium chloride as a moisture absorbent is provided. The sample is placed in the opening of the cup and tightly closed, and allowed to stand in saturated water vapor at 137°C. The water vapor passed through the sample was absorbed into the moisture absorbent (calcium chloride) in the cup, and the mass of the calcium chloride increases. After 24 hours, the mass of the calcium chloride in the cup is measured, and the moisture permeability coefficient is determined based on the increment (moisture amount). The moisture permeability coefficient must be 800 (g.mm/m²·24 hr) or less.

The obtained results are summarized in Table 2, together with the viscosity, tensile shear strength before and after sterilization, and comprehensive evaluation.

### [Table 2]

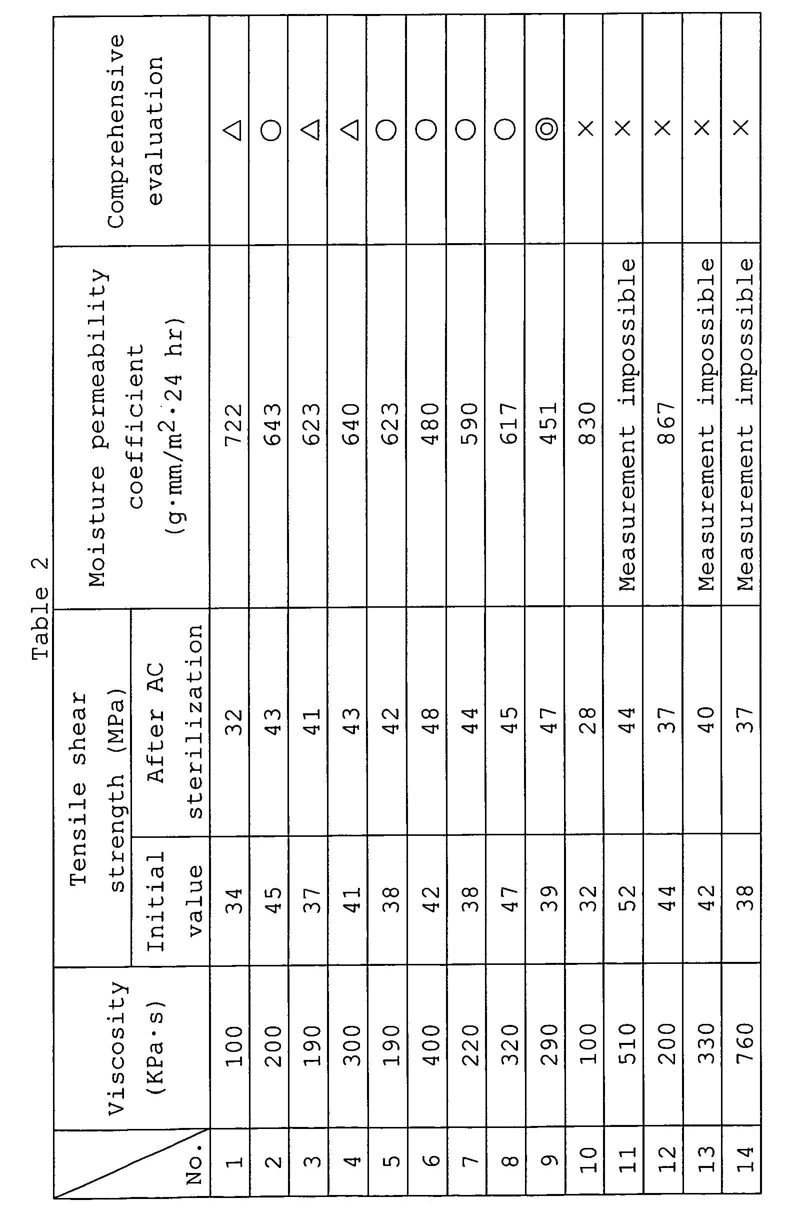

As shown in Table 2, when the spherical fused silica having an average particle size of 0.5 to 20 µm is contained in an amount of 22 to 44% by mass (No. 1 to No. 9), all the viscosity, shear tensile strength, and moisture permeability coefficient reached the acceptable level.

On the other hand, when any of the average particle size, content, or shape of the silica was out of the predetermined range (No. 10 to No. 14), desired properties were not achieved. Specifically, when the silica content was too low (No. 10), the moisture permeability coefficient was more than 800 (g.mm/m²·24 hr) because of the relatively high proportion of the resin. When the silica content was too high (No. 11), the sample was cracked because of its fragility, so that the moisture permeability coefficient could not be measured. In addition, the viscosity of the composition No. 11 was high and more than 100000 mPa·s.

When the silica had a small average particle size (No. 12), the moisture permeability coefficient was more than 800 (g.mm/m²·24 hr), and when the average particle size was large (No. 13), the sample was broken, so that its moisture permeability coefficient could not be measured. When the silica was not spherical silica, the sample was broken even though the conditions of the average particle size and content were fulfilled, so that the moisture permeability coefficient could not be measured, and the viscosity of the composition was more than 100000 mPa·s as shown by the result of No. 14.

If any one of the viscosity, tensile shear strength, and moisture permeability coefficient was out of the predetermined range, the comprehensive evaluation was NG. More specifically, it was confirmed that the adhesive composition cannot achieve the object of the present invention.

The adhesive compositions No. 1 to No. 9 were individually applied to components of an endoscope device, and these components were bonded and cured at 80°C for 2 hours.

As a component of the endoscope device, the end of a flexible envelope tube in the inserting section was wound by a thread from the outside, thereby fixing the tube on the inside member. The adhesive composition was applied to the thread, thereby achieving an outer surface finish. In addition, the imaging device of the endoscope device was sealed using the adhesive composition.

Furthermore, a mound of an adhesive layer was formed using the adhesive composition around the viewing lens and illuminating lens of the endoscope device, thereby smoothing the edges of the outer periphery of the viewing lens and illuminating lens. As described above, the endoscope device was assembled without problem.

The present invention is not limited to the above examples, and may be subjected to various modifications without departing from the scope of the present invention.

In the above-described explanation, the medical device including the adhesive composition is an endoscope device, but the medical device is not particularly limited as long as it is an instrument inserted into or placed in contact with a living body. Examples of the medical device include an endoscope device, surgical device, cell extracting device, blood component centrifuge, and transfusion device. Reference Signs List
- 1: endoscope device
- 2: inserting section
- 3: tip end part
- 4: bending part
- 5: flexible tube
- 7: operating section
- 8: universal cord
- 21: light guide fiber
- 22: imaging unit
- 22a: lens group
- 23: tip hard portion
- 24: tip cover
- 25: adhesive layer
- 31: bending rubber
- 34: thread winding portion
- 36: adhesive layer
- 41: electrical insulation member
- 42: forceps channel
- 43: objective lens frame
- 45: objective lens
- 46: illuminating lens
- 48: septum
- 49: adhesive layer

## Claims

1. An adhesive composition comprising:
a main agent comprising a bisphenol A epoxy resin, a phenol novolac epoxy resin, and an acrylic rubber;
a curing agent comprising xylylenediamine and its derivative; and
a filler, the filler being spherical silica having an average particle size of 0.5 to 20 µm, and accounting for 22 to 44% by mass of a total amount of the adhesive composition.

2. The adhesive composition according to claim 1, wherein the silica is fused silica.

3. The adhesive composition according to claim 2, wherein the fused silica is natural silica.

4. The adhesive composition according to any one of claims 1 to 3, wherein a content of the silica is from 34 to 42% by mass of the total amount of the adhesive composition.

5. The adhesive composition according to any one of claims 1 to 4, wherein a volume average particle size of the silica is from 1 to 10 µm.

6. An endoscope device comprising at least two component members bonded together through an adhesive layer formed by curing the adhesive composition of any one of claims 1 to 5.
